# EUROPEAN PATENT APPLICATION

(11) **EP 4 674 846 A1**
(43) Date of publication of application: **07.01.2026**
(21) Application number: 24185957.8
(22) Date of filing: 02.07.2024
(51) Int. Cl.: C07D 317/72, C07C 273/00, C07D 319/08, C07D 339/08, A61K 31/357, A61K 31/385, A61P 31/04

(54) **UREA MOTIF CONTAINING COMPOUNDS AS ANTIBACTERIAL DRUGS AGAINST MULTIRESISTANT MYCOBACTERIA**

(71) Applicant: Technische Universität München, 80333 München (DE)
(72) Inventor: Sieber, Stephan, 86919 Utting (DE); Mostert, Dietrich, 80798 München (DE); Braun, Josef, 81371 München (DE)
(74) Representative: Dr. Gassner & Partner mbB

(57) **Abstract**

The invention concerns a compound comprising a residue consisting of a first molecular ring and a second molecular ring connected to each other via one common atom which compound has a structure according to formula I 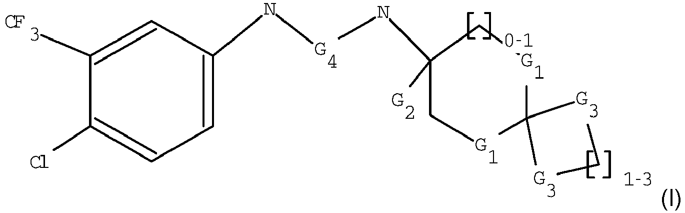 wherein
G₁ is independently selected from a group consisting of C, O, N, and S,
G₂ is independently selected from a group consisting of C and H,
G₃ is independently selected from a group consisting of C, O, N and S and
G₄ is independently selected from a group consisting of C=O, CH₂, C=S and C=NH.

## Description

The invention relates to a compound inhibiting activity of mycobacterial membrane protein Large 3 (MmpL3) and epoxide hydrolases (EpH), a pharmaceutical composition comprising this compound and the compound or the pharmaceutical composition for use as a medicament and in a treatment of a disease. The invention further relates to a kit for preparation of a pharmaceutical composition, a use of a compound according to the invention, and methods for synthesizing a compound according to the invention.

It has been reported, that a total of 1.3 million people died from tuberculosis (TB) in 2022 (https://www.who.int/news-room/fact-sheets/detail/tuberculosis). Worldwide, TB is the second leading infectious killer after COVID-19. Estimated 10.6 million people fell ill with TB worldwide indicating that TB is present in all countries and age groups (Bagcchi, S. WHO's Global Tuberculosis Report 2022. The Lancet Microbe 2023, 4 (1), e20.). TB is usually caused by *Mycobacterium tuberculosis* bacteria.

Strains of *Mycobacteria* represent a significant challenge due to their elaborate and unique cell wall structure, which effectively prevents the penetration of small-molecules. The treatment of *Mycobacterium tuberculosis* infections relies so far only on drugs discovered decades ago comprising first-line antibiotics Rifampicin, Isoniazid (INH) and Ethambutol, all targeting the cell wall biosynthesis of *Mycobacterium tuberculosis.*

MmpL3 is a membrane transporter required for the translocation of trehalose monomycolates (TMM) across the mycobacterial inner membrane, where the mycolic acid chain is transferred to arabinogalactan via the Ag85 complex to yield trehalose dimycolate (TDM). MmpL3 also transports other lipids required to strengthen the cell wall and is therefore regarded as an essential protein and promising drug target. Several inhibitors of MmpL3, such as SQ109, have already been identified via high-throughput screens and rational design campaigns. In addition, carboxamides, benzothiazole amides, pyrrols, benzimidazoles, spiropiperidines, and adamantly ureas (AU1235) have been reported as MmpL3 inhibitors. However, most MmpL3 inhibitors have hydrophobic properties which limits their bioavailability.

Another target class are epoxide hydrolases (EpH), such as EphD or EphF, due to their role in mycolic acid metabolism. EphD was shown to be essential for survival of *Mycobacterium tuberculosis* in macrophages and the biosynthesis of oxygenated mycolic acid species, which are important for maintaining the integrity of the cell envelope.

The rise in the number of multi-resistant strains of *Mycobacterium tuberculosis* is a public health crisis and a health security threat due to limited treatment options.

Multidrug-resistant TB (MDR-TB) refers to a form of TB caused by a strain of *Mycobacterium tuberculosis* that is resistant to rifampicin and isoniazid, used, up to now, as the most effective first-line TB drugs. MDR-TB is treatable by using second-line drugs. However, second-line TB treatment options require extensive medicines that are expensive and toxic due to a daily administration for a duration of six to nine months.

In addition to multidrug resistance, it is known, that an extensively drug-resistant tuberculosis (XDR-TB) can develop, when bacteria do not respond to the most effective second-line TB drugs. XDR-TB is defined as TB caused by a strain of *Mycobacterium tuberculosis* that is resistant to first-line TB drugs rifampicin and/or isoniazid and that is also resistant to any fluoroquinolone, comprising levofloxacin or moxifloxacin, and to at least one other Group A drug. Group A drugs are the most potent group of drugs in the ranking of second-line medicines for treatment of multidrug-resistant TB using longer treatment regimens and comprise levofloxacin, moxifloxacin, bedaquiline and linezolid (https://www.who.int/news/item/27-01-2021-who-announces-updated-definitions-of-extensively-drug-resistant-tuberculosis; World Health Organisation. WHO Consolidated Guidelines on Tuberculosis Module 4: Treatment Drug-Resistant Tuberculosis Treatment. 2020).

The rise in the number of MDR-TB and XDR-TB has significantly impaired the effectiveness of these compounds and draws attention to new therapeutic agents with novel mechanisms of action.

WO 2017/207556 A2 relates to urea motif containing compounds substituted with two aromatic rests. The compounds show anti-bacterial activity against various bacterial strains, in particular against multi-resistant *Staphylococcus aureus* and clinical isolated thereof.

Shao, M. et al., "MmpL3 inhibitors as antituberculosis drugs", European Journal of Medicinal Chemistry 200, 2020, 112390 reveals a compound and their use as a therapeutic agent in preventing mycobacterial infections by inhibition of MmpL3. Said compound contains a NH-C=O group which is substituted with an electron-poor aromatic residue and a spiro compound residue.

Li, M. et al., "Potency increase of spiroketal analogs of membrane inserting indolyl mannich base antimycobacterials is due to acquisition of MmpL3 inhibition", ACS Infect. Dis. 2020, 6, 7, 1882-1893 discloses compounds and their use as therapeutic agent in preventing mycobacterial infections by inhibition of MmpL3, wherein said compounds comprise an electron-poor aromatic residue and a non-aromatic residue consisting of a heterocyclic spiro-compound.

WO 2018/082567 A1 discloses a compound for use in treatment of cancer, wherein said compound is a urea residue substituted with an electron-poor aromatic residue and a non-aromatic residue consisting of a heterocyclic spiro-compound.

Sigmaaldrich.com discloses 1,3-Bis(4-chloro-3-(trifluoromethyl)phenyl)urea (CAS-Number: 370-50-3) which is a urea residue substituted with two electron-poor aromatic residues.

There is still a great need for novel small compounds having antibacterial activity for the treatment of TB, in particular for the treatment of MDR-TB and XDR-TB.

The problem to be solved by the present invention is to provide a new compound and a pharmaceutical composition comprising said compound, said compound and said pharmaceutical composition for a medical use, a kit for preparation of a pharmaceutical composition, a use of said compound and a method for synthesizing it.

The problem is solved by the subject-matter of claims 1, 6, 9, 10 and 12 to 15. Embodiments are subject-matter of claims 2 to 5, 7, 8 and 11.

According to the invention a compound or a pharmaceutically acceptable salt, a solvate or a hydrate of said compound is provided. The compound comprises a residue consisting of a first 5- or 6-membered, in particular non-aromatic, molecular ring and a second 4-, 5- or 6-membered, in particular non-aromatic, molecular ring connected to each other via one common atom - a so called spiro atom - which compound has a structure according to formula (I) 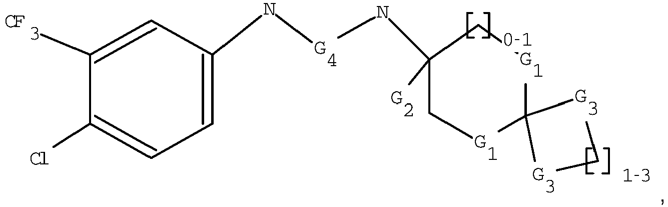 wherein G₁ is independently selected from a group consisting of C, O, N, and S, wherein G₂ is independently selected from a group consisting of C and H, wherein G₃ is independently selected from a group consisting of C, O, N and S and wherein G₄ is independently selected from a group consisting of C=O, CH₂, C=S and C=NH.

In the above formula (I) and all following formula and throughout the entire description, claims and figures, free valences at atoms for which no bound atoms are indicated are understood to be occupied in each case by hydrogen.

The inventors found that the compound according to the invention has unexpected advantageous properties. In particular, it was found that the compound is a potent MmpL3 inhibitor showing improved antibacterial properties and pharmacokinetic profiles. In the present case, the compound exhibit enhanced inhibitory properties towards both MmpL3 and EpH, suggesting a mechanism of action which is unknown up to now. If MmpL3 is blocked, TMM levels increase while TDM levels decline. In experiments under- and over-expressing *Mycobacterium tuberculosis* H37Rv strains have been used to determine minimal inhibitory concentration (MIC) shifts. Surprisingly and in contrast to the known MmpL3 inhibitor SQ109 which shows the expected higher susceptibility of MmpL3 in under-expressing and lower susceptibility in over-expressing strains, the novel compound exhibited higher susceptibility only in under-expressing strains but not when MmpL3 is over-expressed. This suggests a mechanism of action which is unknown up to now. In addition, the compound according to the invention show a significant inhibition of EphF. These novel features could not have been expected. Moreover, the new compound shows a relatively low hydrophobicity and thus an improved bioavailability vis-à-vis known MmpL3 inhibitors.

The compound according to the invention may be a compound, in which G₁ is C or O, G₂ is H, G₃ is C or O and G₄ is C=O, C=S, or C=NH, in particular C=O.

In an embodiment the first molecular ring of the above mentioned residue comprised by the compound is a 6-membered molecular ring and/or the second molecular ring of said residue is a 4-membered molecular ring. Said residue may be a 5,9-dioxaspiro[3.5]nonan-7-yl- or 1,4-dioxaspiro[4.5]decan-8-yl-group.

In the compound according to the invention G₁ may be O, G₂ may be H, G₃ may be C, and G₄ may be C=O or G₁ may be C, G₂ may be H, G₃ may be O, and G₄ may be C=O.

In an embodiment, the compound is selected from the group consisting of 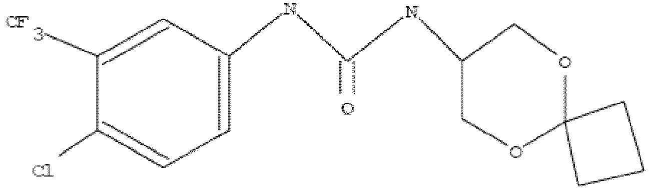 (1-(4-chloro-3-(trifluoromethyl)phenyl)-3-(5,9-dioxaspiro[3.5]nonan-7-yl)urea), and a pharmaceutically acceptable salt, a solvate and a hydrate thereof, 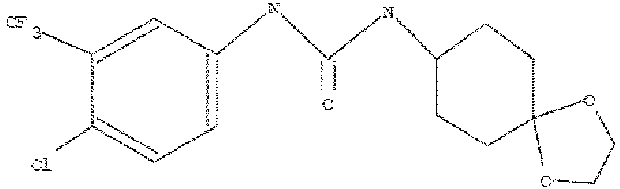 (1-(4-chloro-3-(trifluoromethyl)phenyl)-3-(1,4-dioxaspiro[4.5]decan-8-yl)urea), and a pharmaceutically acceptable salt, a solvate and a hydrate thereof.

As used herein and throughout the entire description, the term "pharmaceutically acceptable salt of a compound" has its usual meaning in the art, i. e. it refers to a salt that retains the desired biological activity of said compound - i. e. in the above case of (1-(4-chloro-3-(trifluoromethyl)phenyl)-3-(5,9-dioxaspiro[3.5]nonan-7-yl)urea) or (1-(4-chloro-3-(trifluoromethyl)phenyl)-3-(1,4-dioxaspiro[4.5]decan-8-yl)urea) - and does not impart any undesired toxicological effects (see e.g., Berge, S. M., et al. (1977) J. Pharm. Sci. 66: 1-19). Examples of such salts are salts of derivatives of the compound with ionizable functional groups which include acid addition salts and base addition salts, in particular acid addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

As used herein and throughout the entire description, the term "pharmaceutically acceptable" may in particular mean approved by a regulatory agency or generally recognized pharmacopoeia for use in animals, and more particularly in humans. The term "solvate" as used herein refers to an addition complex of a dissolved material in a solvent (such as an organic solvent (e.g., an aliphatic alcohol (such as methanol, ethanol, n-propanol, isopropanol), acetone, acetonitrile, ether, and the like), water or a mixture of two or more of these liquids), wherein the addition complex exists in the form of a crystal or mixed crystal. The amount of solvent contained in the addition complex may be stoichiometric or non-stoichiometric. A "hydrate" is a solvate wherein the solvent is water.

The present invention also relates to a pharmaceutical composition comprising the compound according to the invention and a pharmaceutically acceptable excipient.

The pharmaceutically acceptable excipient may be a liquid such as water or a physiological salt solution. If the excipient is the physiological salt solution the composition may be applied by injection, in particular subcutaneous or intravenous injection. For oral application the excipient may be, for example a sugar such as glucose or saccharose. Suitable forms of preparations include spray formulations, patches, tablets, capsules, dragées, lozenges, powders, granules, powders, or liquid preparations for example, suspensions, solutions, emulsions, or syrups.

In a specific embodiment, the pharmaceutical composition according to the invention comprises at least one additional pharmaceutical agent, in particular an antituberculosis agent, for example selected from the group consisting of isoniazid, rifampicin, pyrazinamide, ethambutol, streptomycin, kanamycin, amikacin, capreomycin, ofloxacin, levofloxacin, moxifloxacin, cycloserine, para-aminosalicylic acid, ethioamide, prothionamide, thioacetazone, clofacimine, amoxicillin with clavulanate, imipenem, linezolid, clarithromycin, and thioridazine.

The invention further concerns the compound or the pharmaceutical composition according to the invention for use as a medicament.

The invention also concerns the compound or the pharmaceutical composition according to the invention for use in a method for the treatment of a bacterial disease.

The compound or pharmaceutical compositions according to the invention for use as a medicament or in the method for the treatment of a bacterial disease may be administered in either a single dose or multiple doses and may be prepared for a suitable administration route including but not limited to nasal, inhalation, topical, oral, oral mucosal, rectal, pleural, intraperitoneal, intramuscular, subcutaneous, transdermal, epidural, intrathecal, and intravenous administration. Injection may be a depot injection, i.e. injection of a localized mass of the compound according to the invention, called a depot, from which depot the compound is gradually absorbed by surrounding tissue. The treatment may also occur by infiltration with the compound of the invention. Infiltration involves loading a volume of tissue with the compound of the invention by injection thus filling the interstitial space of that tissue with the compound.

If the compound or the pharmaceutical composition according to the invention is for use in a method for treatment of a bacterial disease, the bacterial disease may be caused by at least one bacteria selected from but not limited to the group of gram-positive bacteria consisting of *Mycobacterium tuberculosis, multidrug-resistant Mycobacterium tuberculosis, extensively drug-resistant Mycobacterium tuberculosis, bedaquiline-resistant Mycobacterium tuberculosis and clinical isolates thereof, Mycobacterium bovis, Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium, intracellulare, Mycobacterium simiae, Mycobacterium szulgai, Mycobacterium xenopi, Mycobacterium fortuitum, Mycobacterium chelonei, Mycobacterium marinum.* The compound or the pharmaceutical composition for use according to the invention has been found to be particularly effective against *multidrug resistant* and *extensively drug-resistant Mycobacteria.*

A further aspect of the invention concerns a kit for preparation of a pharmaceutical composition comprising the compound according to the invention and at least one pharmaceutically acceptable carrier.

As used herein and throughout the entire description, the terms "carrier" and "excipient" are used interchangeably herein. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO2), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti-oxidants (e.g. BHT, BHA, Ascorbic acid), wetting agents (e.g. polysorbates, sorbitan esters), anti-foaming agents (e.g. Simethicone), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). The person skilled in the art will readily be able to choose suitable pharmaceutically acceptable carriers or excipients, depending, e.g., on the formulation and administration route of the pharmaceutical composition.

The invention further concerns a use of a compound according to the invention as a disinfectant, wherein any method for treatment of the human or animal body by surgery or therapy is excluded.

Another aspect of the invention concerns a method for synthesis 1-(4-chloro-3-(trifluoromethyl)phenyl)-3-(5,9-dioxaspiro[3.5]nonan-7-yl)urea comprising at least the following steps:
a) Dissolution of 5,9-dioxaspiro[3.5]nonan-7-amine in a polar aprotic solvent,
b) addition of 1-chloro-4-isocyanato-2-(trifluoromethyl)benzene,
c) stirring of the resulting solution
and either
d1) addition of water and separation of aqueous and organic phase,
e1) washing of the organic phase with water and removal of volatile components of the remaining organic phase by vacuum evaporation until a solid residue is obtained,
f1) dissolution of the residue in a polar aprotic solvent having a dielectric constant of at least 30 at 25 °C, in particular in dimethylformamide or dimethyl sulfoxide (DMSO), and precipitation by addition of water,
g1) filtering of a precipitate obtained in step f) and removal of residual water and
h1) performing a chromatography for obtaining a fraction containing 1 -(4-chloro-3-(trifluoromethyl)phenyl)-3-(5,9-dioxaspiro[3.5]nonan-7-yl)urea in an eluent and optionally removing the eluent for obtaining 1-(4-chloro-3-(trifluoromethyl)phenyl)-3-(5,9-dioxaspiro[3.5]nonan-7-yl)urea as a solid product
   or
d2) removal of the solvent from the solution and
e2) performing a chromatography for obtaining a fraction containing 1-(4-chloro-3-(trifluoromethyl)phenyl)-3-(5,9-dioxaspiro[3.5]nonan-7-yl)urea in an eluent and optionally removing the eluent for obtaining 1-(4-chloro-3-(trifluoromethyl)phenyl)-3-(5,9-dioxaspiro[3.5]nonan-7-yl)urea as a solid product.

The solvent of step a) is a polar aprotic solvent. An aprotic solvent is a solvent that is not able to donate a proton, i. e. the molecules forming the solvent do not dissociate in a proton and a remaining residue. In other words, it is a solvent that lacks an acidic proton. A polar solvent is understood as a solvent having a dielectric constant of 6 or above, in particular of 8.8 or above, at 25 °C. The solvent of step a) may be a solvent that lacks hydroxyl and amine groups. The solvent of step a) may comprise at least one of acetone, acetonitrile, dichloromethane (DCM), in particular dry DCM, dimethylformamide (DMF), dimethylpropyleneurea, dimethyl sulfoxide, ethyl acetate, hexamethylphosphoramide, pyridine or tetrahydrofuran or any other solvent of Table 1. Dry DCM herein was purchased from Thermo Fisher Scientific and is DCM having a water content of at most 0.005 % determined by coulometry.

**Table 1:**

| **Solvent** | **Chemical formula** | **Dielectric constant at 25 °C** |
|---|---|---|
| acetone | C₃H₆O | 20.7 |
| acetonitrile | CH₃CN | 37.5 |
| dichloromethane | CH₂Cl₂ | 8.93 |
| dimethylformamide | (CH₃)₂NCHO | 36.7 |
| dimethylpropyleneurea | (CH₃)₂C₄H₆N₂O | 36.12 |
| dimethyl sulfoxide | (CH₃)₂SO | 46.7 |
| ethyl acetate | C₄H₈O₂ | 6.02 |
| hexamethylphosphoramide | [(CH₃)₂N]₃PO | 29.6 |
| pyridine | C₅H₅N | 12.4 |
| tetrahydrofuran | C₄H₈O | 7.6 |
| sulfolane | C₄H₈O₂S | 43.34 |

Step c) may be performed for 1 to 48 hours, in particular for 10 to 40 hours, in particular for 12 to 20 hours, in particular for 14 hours. Temperatures at step c) may be in the range of 20 to 25 °C, in particular in the range of 21 to 24 °C, in particular 22 °C.

Vacuum evaporation of step e1) may be performed under a pressure reduced vis-à-vis atmospheric pressure, e. g. at a pressure in the range of 5 to 950 mbar at 40 °C.

The solvent in step f1) may be dimethylformamide.

The removal of residual water at step g1) and of the solvent at step d2) may be achieved by any known method in the art, e. g. by co-evaporation with methanol or ethanol, by use of a desiccator or by freeze drying. In particular, it is achieved by co-evaporation with methanol.

The solid product obtained by performing the chromatography and removing the eluent in step h1) or e2) may be a white solid product. The chromatography may be a column chromatography, in particular a flash chromatography. The chromatography in step h1) or e2) may be performed on a silica column as stationary phase with a mixture of DCM and methanol, in particular DCM and methanol in a DCM:methanol volume ratio of 98:2, as mobile phase, i. e. as eluent. Alternatively, an eluent with similar polarity, i. e. an eluent having a dielectric constant at 25 °C of +/- 10 of that of the mixture of DCM and methanol, in particular DCM and methanol in the DCM:methanol volume ratio of 98:2, can be used. Aluminum oxide or a reversed phase material may also be used as stationary phase. In case of aluminum oxide as stationary phase the eluent may also be a mixture of DCM and methanol. The reversed phase material may consist of porous silica-gel particles to the surfaces of which hydrocarbons, such as C3, C4, C8, C18 or more, are chemically bonded. Appropriate eluents depend on the stationary phase. In case of a reverse phase material as stationary phase the eluent is a polar solvent, such as a mixture of water and a polar organic solvent, such as methanol or acetonitrile. For example, the eluent may be a water - acetonitrile gradient from 30 to 100 v/v % of acetonitrile. Fractions containing the product can be identified, e. g., by thin layer chromatography (TLC), liquid chromatography-mass spectrometry (LC-MS) or Nuclear magnetic resonance (NMR) spectroscopy.

A further aspect of the invention concerns a method for synthesis of 1-(4-chloro-3-(trifluoromethyl)phenyl)-3-(1,4-dioxaspiro[4.5]decan-8-yl)urea comprising at least the following steps:
a) Dissolution of 1-chloro-4-isocyanato-2-(trifluoromethyl)benzene in a polar aprotic solvent,
b) addition of 1,4-dioxaspiro[4.5]decan-8-amine,
c) stirring of the resulting solution
and either
d1) filtering of a precipitate formed in step c) and washing of the precipitate, in particular with the/a further polar aprotic solvent, in particular with DCM or DMF, for obtaining 1-(4-chloro-3-(trifluoromethyl)phenyl)-3-(1,4-dioxaspiro[4.5]decan-8-yl)urea as a solid product
   or
d2) removal of the solvent from the solution and
e2) performing a chromatography for obtaining a fraction containing 1-(4-chloro-3-(trifluoromethyl)phenyl)-3-(1,4-dioxaspiro[4.5]decan-8-yl)urea in an eluent and optionally removing the eluent for obtaining 1-(4-chloro-3-(trifluoromethyl)phenyl)-3-(1,4-dioxaspiro[4.5]decan-8-yl)urea as a solid product.

The polar aprotic solvent of step a) and of step d1) may be independently form each other a solvent as described above for the synthesis of 1-(4-chloro-3-(trifluoromethyl)phenyl)-3-(5,9-dioxaspiro[3.5]nonan-7-yl)urea. In particular it may be dry dichloromethane (DCM), i. e. DCM having a water content of at most 0.005 % determined by coulometry.

Step c) may be performed for 10 to 40 hours, in particular for 12 to 30 hours, in particular for 12 to 20 hours, in particular for 14 hours. Temperatures of step c) may be in the range of 20 to 25 °C, in particular in the range of 21 to 24 °C, in particular 22 °C.

Removal of the solvent at step d2) may be performed as described above for the synthesis of 1-(4-chloro-3-(trifluoromethyl)phenyl)-3-(5,9-dioxaspiro[3.5]nonan-7-yl)urea. The chromatography at step e2) may be performed as described above for steps h1) or e2) of the method for synthesis of 1-(4-chloro-3-(trifluoromethyl)phenyl)-3-(5,9-dioxaspiro[3.5]nonan-7-yl)urea. The chromatography may be a column chromatography, in particular a flash chromatography.

The solid product obtained in step d1) or step e2) may be a rose powder.

In a specific embodiment, the polar aprotic solvent is DCM, in particular dry DCM.

In the above methods comprising chromatography, removal of the eluent may be performed as described above for the removal of the solvent at step d2) of the synthesis of 1-(4-chloro-3-(trifluoromethyl)phenyl)-3-(5,9-dioxaspiro[3.5]nonan-7-yl)urea, i. e. it may be achieved by any known method in the art, e. g. by co-evaporation with methanol or ethanol, by use of a desiccator or by freeze drying and in particular by co-evaporation with methanol.

All features indicated in the specification are to be understood as features applicable to all embodiments of the invention. This means, for example, that a feature indicated for the composition according to the invention can also be applied to the method according to the invention, and/or the use according to the invention, and vice versa.

The invention shall be explained in more detail by the following examples.

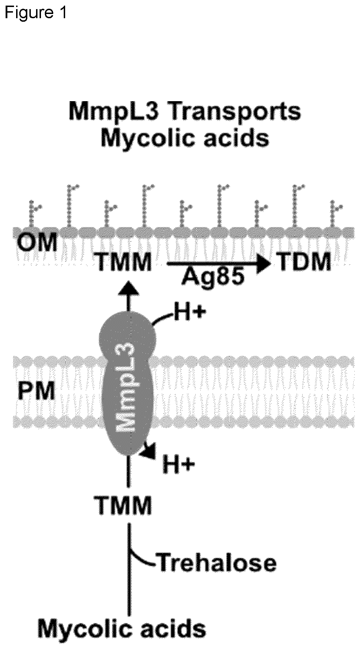

Figure 1 shows schematically the export of trehalose monomycolate (TMM) across the mycomembrane (PM) by the mycolic acid transporter MmpL3. TMM is then converted to trehalose dimycolate (TDM) by Ag85 before being incorporated into the outer membrane (OM) of *Mycobacteria.*

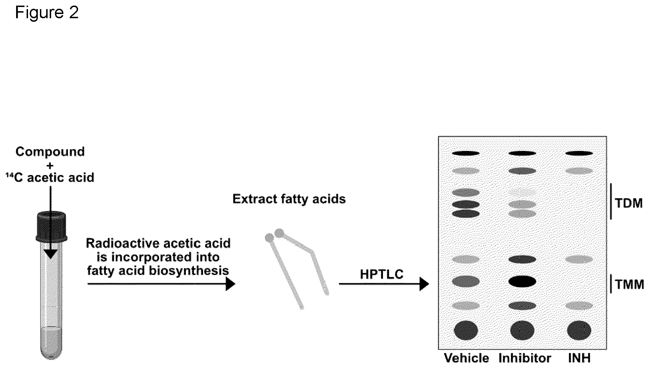

Figure 2 shows the MmpL3 activity by measurement in living cells after feeding ¹⁴C acetic acid, which is incorporated into mycolic acids. After the extraction of fatty acids and high performance thin layer chromatography (HPTLC), their relative abundance is measured by autoradiography. As TMM is only converted to TDM after export by MmpL3, the ratio of TDM to TMM is a direct measure of MmpL3 activity. Mycolic acids are identified by comparison to cells treated with isoniazid (INH). INH completely inhibited mycolic acid biosynthesis.
Figure 3 is a diagram showing the dose-response curves of *Mycobacterium tuberculosis* H37Rv strain, WT, MmpL3 over- (+ATc), and underexpressing (-ATc) strains dosed with different concentrations of compounds 227 or SQ109.
Figure 4 is a diagram showing the MmpL3 activity in *Mycobacterium tuberculosis* H37Ra cells treated with different concentrations of the compound according to the invention and SQ109.
Figure 5 is a bar diagram showing the EphF activity of purified EphF treated with different concentrations of compound 227 and 21.
Figure 6 is a diagram showing the efficacy of compound 21 in PK studies.
Figure 7 is a diagram showing the efficacy of compound 22 in PK studies.
Figure 8 is a diagram showing the dose escalation study of compound 21 according to the invention.

### 1. Material and methods

### 1.1 Reagents

Reagents and solvents were purchased from commercial suppliers (Thermo Fisher Scientific Inc., Merck KGaA, Alfa Aesar GmbH, Carl Roth GmbH + Co. KG, VWR International GmbH, Enamine Germany GmbH). SQ109 was commercially purchased from Medchemexpress (https://www.medchemexpress.com/SQ109.html?locale=de-DE). All reagents and solvents were used as purchased without further purification.

### 1.2 Cell culture

Cell culture media and supplements were obtained from commercial suppliers (Sigma-Aldrich^{®}, Merck KGaA, Sarstedt). Human epitheloid cervix carcinoma (HeLa) cells were purchased from Sigma Aldrich^{®}, Merck KGaA (93021013) and were cultured in Dulbecco's Modified Eagle Medium (DMEM) comprising 4.5 g/L glucose, supplemented with 10 % fetal bovine serum (FBS) (Sigma-Aldrich^{®}, Merck KGaA) and 2 mM L-glutamine (Sigma-Aldrich^{®}, Merck KGaA) in T-175 culture flask (Sarstedt). Cells were maintained in a humidified 37 °C incubator with 5 % CO₂. Cells were detached with Accutase^{®} (Sigma-Aldrich^{®}, Merck KGaA).

### 1.3 Bacterial strains and media

*Mycobacteria* were cultured in 7H9+OADC+0.05% Tween-80. OADC (0.5 g/L oleic acid, 50 g/L BSA Frac V, 20 g/L Dextrose, 30 mg/L catalase) was sterile filtered and added 1:10 to autoclaved 7H9 broth (4.7 g/L 7H9 powder). The broth was stored in the fridge for no more than 4 weeks. For each culture, sterile filtered 20% Tween-80 was added freshly to a final concentration of 0.05%. *Mycobacterium tuberculosis* H37Ra (purchased from ATCC, ATCC-25177) was inoculated 1:100 from a glycerol Stock and grown for 10-21 days in T-175 cell culture flasks (37 °C, humidified) prior to any experiment. *Mycobacterium smegmatis* DSM43756 (purchased from DSMZ (German Collection of Microorganisms and Cell Cultures)) was inoculated 1:1000 from a glycerol stock and grown for 3 days prior to experiments. *Mycobacterium tuberculosis* H37Ra was grown at 37 °C in a humidified plate incubator, while *Mycobacterium smegmatis* DSM43756 was grown at 37 °C and 200 rpm shaking in Erlenmeyer flasks. All sample preparations for LC-MS based analysis were performed in at least four biological replicates (n=4) from four separately cultured mycobacterial cultures.

### 2. Synthesis of urea motif containing compounds

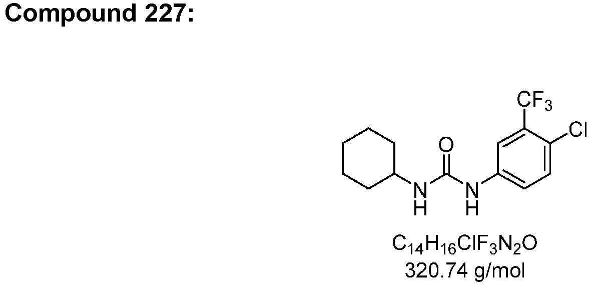

1-(4-chloro-3-(trifluoromethyl)phenyl)-3-cyclohexylurea

Cyclohexylamine (175 µL, 150 mg, 1.51 mmol, 1.1 eq.) was dissolved in dry DCM (10 mL) and 1-chloro-4-isocyanato-2-(trifluoromethyl)benzene (305 mg, 1.37 mmol, 1.0 eq.) was added. The solution was stirred overnight at room temperature. The precipitate was filtered of, rinsed with cold DCM and dried under reduced pressure to yield the product (195 mg, 606 µmol, 44%) as a white solid.

**¹H-NMR** (400 MHz, DMSO-ds): delta [ppm] = 8.78 (s, 1H), 8.06 (d, *J* = 2.2 Hz, 1H), 7.57-7.45 (m, 2H), 6.24 (d, *J* = 7.8 Hz, 1H), 3.52-3.43 (m, 1H, overlapping with the residual water signal), 1.85-1.75 (m, 2H), 1.70-1.61 (m, 2H), 1.58-1.49 (m, 1H), 1.36-1.24 (m, 2H), 1.24-1.11 (m, 3H).

**¹³C-NMR** (101 MHz, DMSO-ds): delta [ppm] = 154.1, 140.2, 131.9, 126.6 (q, J = 30.4 Hz), 124.3, 122.6, 121.3, 116.0 (q, *J* = 5.8 Hz), 47. 9, 32.8, 25.2, 24.4.

**ESI-HR-MS** (m/z) (M-H⁺) calcd. for C₁₄H₁₅ClF₃N₂O⁻, 319.0830; found, 319.0830. 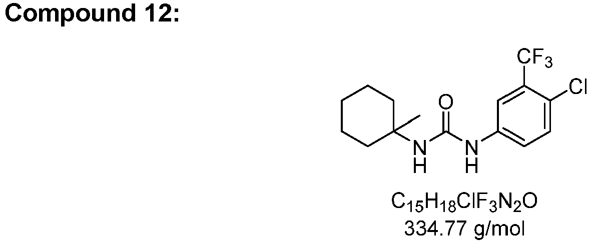 1-(4-chloro-3-(trifluoromethyl)phenyl)-3-(1-methylcyclohexyl)urea

1-Chloro-4-isocyanato-2-(trifluoromethyl)benzene (489mg, 2.21 mmol, 1.0 eq.) was dissolved in DCM (10 mL). Diisopropylethylamine (422 µl, 313 mg, 2.43 mmol, 1.1 eq.) and 1-methylcyclohexan-1-amine hydrochloride (330 mg, 2.20 mmol, 1.0 eq.) were added and the solution was stirred overnight at room temperature. The volatile components were removed under reduced pressure, and the residue was purified by flash chromatography (hexane/ethyl acetate = 85/15). The product was dissolved in DMF (50 mL) and precipitated by adding water. The precipitate was filtered of to yield the product (431 mg, 1.29 mmol, 58%) as a white solid.

**TLC** (hexane/ethyl acetate = 85/15): R*_{f}* = 0.31 [UV]

**¹H-NMR** (400 MHz, DMSO-ds): delta [ppm] = 8.80 (s, 1H), 8.06 (d, *J* = 2.5 Hz, 1H), 7.51 (d, *J* = 8.7 Hz, 1H), 7.44 (dd, J = 8.8, 2.5 Hz, 1H), 5.94 (s, 1H), 1.99-1.88 (m, 2H), 1.49-1.39 (m, 5H), 1.32-1.18 (m, 6H).

**¹³C-NMR** (101 MHz, DMSO-ds): delta [ppm] = 153.8, 140.2, 131.8, 126.6 (q, *J* = 30.4 Hz), 124.2, 121.9, 121.1-120.7 (m), 115.6 (q, *J* = 5.8 Hz), 51.6, 36.4, 26.9, 25.2, 21.5.

**ESI-HR-MS** (m/z) (M-H⁺) calcd. for C₁₅H₁₇ClF₃N₂O⁻, 333.0987; found, 333.0987. 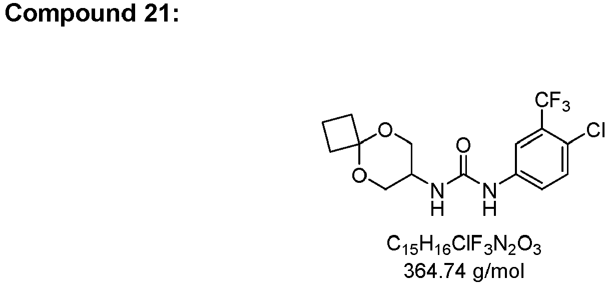 1-(4-chloro-3-(trifluoromethyl)phenyl)-3-(5,9-dioxaspiro[3.5]nonan-7-yl)urea

5,9-Dioxaspiro[3.5]nonan-7-amine (200 mg, 1.40 mmol, 1.0 eq.) was dissolved in dry DCM (10 mL), and 1-chloro-4-isocyanato-2-(trifluoromethyl)benzene (309 mg, 1.40 mmol, 1.0 eq.) was added. The solution was stirred overnight at room temperature. Water (25 mL) was added, and the phases were separated. The organic phase was washed with water (25 mL), and the volatile components were removed under reduced pressure. The residue was dissolved in dimethylformamide (5 mL) and precipitated with water (25 mL). The precipitate was filtered of and residual water was removed by co-evaporation with methanol (MeOH). Flash chromatography (DCM/MeOH = 98/2) yielded the product (261 mg, 718 µmol, 51%) as a white solid.

**Yield:** 51% (261 mg, 718 µmol); workup A (DCM/MeOH = 98/2).

TLC (DCM/MeOH = 97/3): R*_{f}* = 0.30 [UV]

**¹H-NMR** (400 MHz, DMSO-ds): delta [ppm] = 9.12 (s, 1H), 8.06 (s, 1H), 7.61-7.45 (m, 2H), 6.70 (d, J = 7.9 Hz, 1H), 3.96-3.86 (m, 2H), 3.65-3.51 (m, 3H), 2.25-2.09 (m, 4H), 1.72-1.57 (m, 2H).

**¹³C-NMR** (101 MHz, DMSO-ds): delta [ppm] = 154.4, 139.9, 131.9, 126.7 (q, J = 30.4 Hz), 124.2, 122.3, 122.0-121.3 (m), 116.0 (q, J = 5.8 Hz), 101.0, 63.8, 43.4, 33.3, 30.5, 11.1.

**ESI-HR-MS** (m/z) (M-H⁺) calcd. for C₁₅H₁₅ClF₃N₂O₃⁻, 363.0729; found, 363.0728. 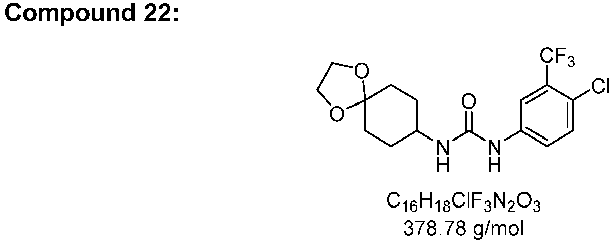 1-(4-chloro-3-(trifluoromethyl)phenyl)-3-(1,4-dioxaspiro[4.5]decan-8-yl)urea

1-Chloro-4-isocyanato-2-(trifluoromethyl)benzene (352 mg, 1.59 mmol, 1.0 eq.) was dissolved in dry DCM (10 mL) and 1,4-dioxaspiro[4.5]decan-8-amine (250 mg, 1.59 mmol, 1.0 eq.) was added. The solution was stirred overnight at room temperature. The precipitate was filtered of and washed with DCM to yield the product (521 mg, 1.37 mmol, 87%) as a slightly rose powder.

**Yield:** 87% (521 mg, 1.37 mmol); workup B.

**¹H-NMR** (400 MHz, DMSO-ds): delta [ppm] = 8.75 (s, 1H), 8.06 (d, J = 1.7 Hz, 1H), 7.68-7.32 (m, 2H), 6.35 (d, J = 7.7 Hz, 1H), 3.85 (s, 4H), 3.65-3.49 (m, 1H), 1.82-1.74 (m, 2H), 1.70-1.61 (m, 2H), 1.59-1.51 (m, 2H), 1.50-1.39 (m, 2H).

**¹³C-NMR** (101 MHz, DMSO-ds): delta [ppm] = 154.2, 140.1, 131.8, 126.6 (q, J = 30.5 Hz), 124.2, 122.2, 121.5-121.1 (m), 116.0 (q, J = 5.6 Hz), 107.3, 63.7, 46.4, 32.3, 29.5.

**ESI-HR-MS** (m/z) (M-H⁺) calcd. for C₁₆H₁₇ClF₃N₂O₃⁻, 377.0885; found, 377.0885.

### 1-(4-chloro-3-(trifluoromethyl)phenyl)-3-(5,9-dithiospiro[3.5]nonan-7-yl)urea:

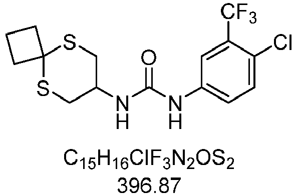

5,9-Dithiospiro[3.5]nonan-7-amine was dissolved in dry DCM (10 mL), and 1-chloro-4-isocyanato-2-(trifluoromethyl)benzene was added. The solution was stirred overnight at room temperature. Water (25 mL) was added, and the phases were separated. The organic phase was washed with water (25 mL), and the volatile components were removed under reduced pressure. The residue was dissolved in dimethylformamide (5 mL) and precipitated with water (25 mL). The precipitate was filtered of and residual water was removed by co-evaporation with methanol (MeOH). Flash chromatography yielded the product.

### 1-(4-chloro-3-(trifluoromethyl)phenyl)-3-(spiro[3.5]nonan-7-yl)urea:

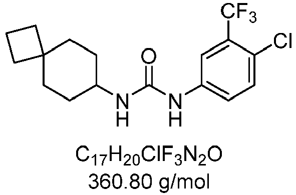

Spiro[3.5]nonan-7-amine was dissolved in dry DCM (10 mL), and 1-chloro-4-isocyanato-2-(trifluoromethyl)benzene was added. The solution was stirred overnight at room temperature. Water (25 mL) was added, and the phases were separated. The organic phase was washed with water (25 mL), and the volatile components were removed under reduced pressure. The residue was dissolved in dimethylformamide (5 mL) and precipitated with water (25 mL). The precipitate was filtered of and residual water was removed by co-evaporation with methanol (MeOH). Flash chromatography yielded the product.

### 1-(4-chloro-3-(trifluoromethyl)phenyl)-3-(spiro[4.5]decan-8-yl)urea:

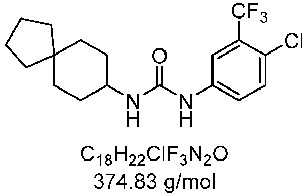

1-Chloro-4-isocyanato-2-(trifluoromethyl)benzene was dissolved in dry DCM (10 mL) and spiro[4.5]decan-8-amine was added. The solution was stirred overnight at room temperature. The precipitate was filtered of and washed with DCM to yield the product.

Throughout the specification, the term "room temperature" means 20 °C, the term "overnight" means a range of 10 to 14 hours and the term "reduced pressure" means a pressure below atmospheric pressure, if not specified otherwise.

### 3. Biological and pharmacological tests

### 3.1 Cytotoxicity assay (MTT)

The MTT assay was performed in transparent, flat-bottomed 96 well plates. HeLa cells were seeded with 4000 cells per well in a volume of 200 µL per well. Cells were grown overnight at 37 °C and 5 % CO₂ to allow the cells to adhere to the surface. The medium was removed and replaced by fresh medium supplemented with respective compound in concentrations ranging from 2 µM to 500 µM and a final DMSO concentration of less than 1 % DMSO or 1 % DMSO as a control. The cells were incubated at 37 °C and 5 % CO₂ for 24 h. For the determination of the metabolic activity, 20 µL of 3-(4,5-dimethyl-2-thiazolyl)-2,5-diphenyl-2H-tetrazolium bromide (MTT) solution (5mg/mL in PBS (8 g/L NaCl, 0.2 g/L KCl, 1.44 g/L, Na₂HPO₄, 0.245 g/L KH₂PO₄)) were added to the cells and the cells were incubated at 37 °C, 5 % CO₂ for 4 h. The medium was removed and the formazan crystals were dissolved in 200 µL DMSO per well under shaking at 300 rpm for 25 min. Optical density was measured at 570 nm and background subtracted at 630 nm using an Infinite F200 pro plate reader (TECAN Infinite M200 Pro). Four biological replicates with 3 technical replicates were measured for respective data point. The cell viability was normalized to DMSO control and graph was fitted as log(inhibitor) vs. response variable slope (four parameters) non-linear regression using GraphPad Prism (version 10.01). Cytotoxicity was reported as IC50 value, i. e. the concentration at which 50 % viability of the cells is reached.

### 3.2 Minimal inhibitory concentration (MIC)

Minimal inhibitory concentrations (MICs) represent the lowest concentration of the sample that will inhibit the visible growth of a microorganism after overnight incubation, and was obtained by a 96 well plate-based assay with serial dilutions of the probes to be tested. In the case of the inoculum of Mycobacteria, a 10 to 14 days culture of *Mycobacterium tuberculosis* H37Ra was harvested by centrifugation at 7000 x g and reconstituted in fresh 7H9+OADC+0.05% Tween-80. Five sterile glass beads were added and culture was homogenized. The culture was incubated for 10 to 20 minutes at room temperature (21 °C) to allow the larger cell clumps to settle at the bottom. The homogenous supernatant was transferred to a sterile falcon tube and the optical density (OD₆₀₀) was measured. The cultures were diluted using the respective medium to an OD₆₀₀ = 0.0063, equivalent to 0.05 McFarland. For the MIC experiments, respective compounds were serially diluted in respective medium, i.e. compounds from a DMSO stock (2 µL) were diluted with 100 µL of medium (1:50) and the serial dilution was prepared on the same plate (50 µL of the dilution + 50 µL of the medium) obtaining various concentrations in the respective wells. Diluted bacterial cultures (50 µL) were added to various concentrations of the respective compound (50 µL). A growth control containing DMSO (50 µL) and cultivated medium (50 µL) and a sterile control containing fresh medium (100 µL) were run on every 96 well plate in triplicates. After incubation of plates with gas-permeable seals for 18 days at 37 °C in a humidified plate incubator, the dilution series was analyzed for microbial growth by measurement of the absorption at 600 nm by an Infinite F200 pro plate reader (TECAN Infinite M200 Pro). MIC values were determined by three independent experiments with at least triplicate runs for each concentration.

### 3.3 Pharmacokinetic properties

For pharmacokinetic experiments CD-1 female mice (22-25 g) were used. All animal experiments were ethically reviewed and carried out in accordance with European Directive 2010/63/EU and the GSK Policy on the Care, Welfare and Treatment of Animals.

Oral snapshot pharmacokinetic (PK) profiling was performed by an oral gavage for administration of the respective compounds at a dose of 25 mg/kg in vehicle consisting of 5% Dimethylacetamice (DMA), and 95% (20% Solutol HS15 in water). Blood samples at a volume of 30 to 40µL were taken by puncture of the lateral tail vein from mice (n = 2 per compound) at 30 minutes, 1, 3, and 5 hour(s) post-dose.

Intravenous (IV) PK was performed at a dose of 5mg/kg in 4% Cremophor^{®} EL (Merck KGaA). Blood samples were taken from mice (n = 3 per route and dose) at 5 minutes, 15 minutes, 1, 3, 7, and 24 hours post dose.

Dose escalation oral PK profiling of compound 21 formulated in a suspension with 20% Solutol HS15 or in solution with 100% PEG400 vehicle was performed after 3 doses of 50, 100, and 200 mg/kg and 400 mg/kg once a day. Blood samples were taken from mice (n = 3 per route and dose) at 30 minutes, 1, 3, 5, 7, and 24 hours post dose.

Blood samples were captured in CB300 blood collection tubes containing K₂EDTA and stored on ice. Plasma was recovered after centrifugation and stored at -80°C until analyzed by high pressure liquid chromatography coupled to tandem mass spectrometry. PK parameters were determined using non-compartmental pharmacokinetic analysis supported by the PK Solver Excel add-in.

### 3.3.1 LC-MS/MS analytical methods for PK studies

For LC-MS/MS analysis, 1mg/mL DMSO stock of compounds were serially diluted in 50/50 Acetonitrile (ACN)/ Milli-Q water to create standard curves solutions. Standards were created by adding spiking solutions (10 µL) to drug free plasma (90 µL) (CD-1 K₂EDTA Mouse, Bioreclamation IVT). The sample to be tested (10 µL of control, standard, or study sample) were added to ACN protein precipitation solvent (100 µL) containing the internal standards Labetalol (100 ng/mL) (Sigma Aldrich). Extracts were vortexed for 5 minutes and centrifuged at 4000 rpm for 5 minutes. The supernatant (75 µL) was transferred for HPLC-MS/MS analysis and diluted with Milli-Q deionized water (75 µL).

LC-MS/MS analysis was performed on a Sciex Applied Biosystems Qtrap 6500+ triple-quadrupole mass spectrometer coupled to a Shimadzu Nexera X2 UHPLC system to quantify the respective drug in plasma. Chromatography was performed on an Agilent SB-C8 (2.1x30 mm; particle size, 3.5µm) using a reverse phase gradient. Milli-Q deionized water with 0.1% formic acid was used for the aqueous mobile phase and 0.1% formic acid in ACN for the organic mobile phase. Multiple-reaction monitoring of parent/daughter transitions in electrospray negative-ionization mode was used to quantify the analytes.

Multiple reaction monitoring (MRM) transitions were used for 227 (319.98/194.40), 21 (362.96/193.90), 22 (377.02/193.80), and Labetalol (327.20/176.00).

Sample analysis was accepted if the concentrations of the quality control samples were within 20% of the nominal concentration. Data processing was performed using Analyst software (version 1.6.2; Applied Biosystems Sciex).

### 4. Compounds and their activities

Compounds 12 and 227 are used as comparative compounds to the compounds according to the invention.

Compound SQ109 is N1-(Adamantan-2-yl)-N2-[(2E)-3,7-dimethylocta-2,6-dien-1-yl]ethane-1,2-diamine and is used as a comparative compound to the compounds according to the invention. 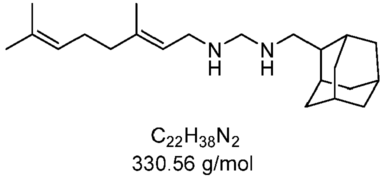 N1-(Adamantan-2-yl)-N2-[(2E)-3,7-dimethylocta-2,6-dien-1-yl]ethane-1,2-diamine

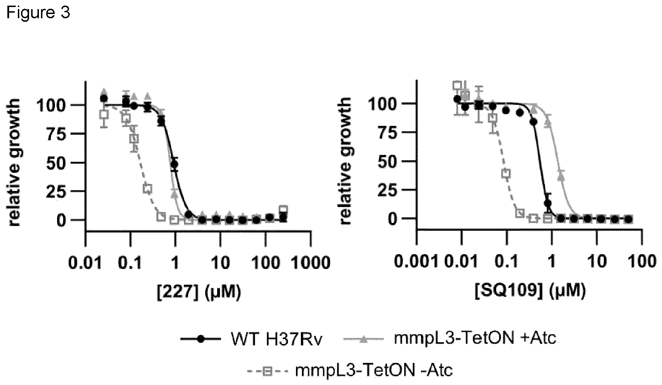

Experiments with MmpL3 under- and over-expressing *Mycobacterium tuberculosis* H37Rv strains were used to determine MIC shifts with the compounds. SQ109 was used as a positive control and shows the expected higher susceptibility of MmpL3 under-expressing and lower susceptibility in over-expressing strains Interestingly, the urea analogues, including compound 227, exhibited higher susceptibility only in under-expressing strains but not when MmpL3 is over-expressed, suggesting a diverging mechanism of action compared to SQ109. Each diagram represents one of two biological replicates, each consisting of three technical replicates (Figure 3).

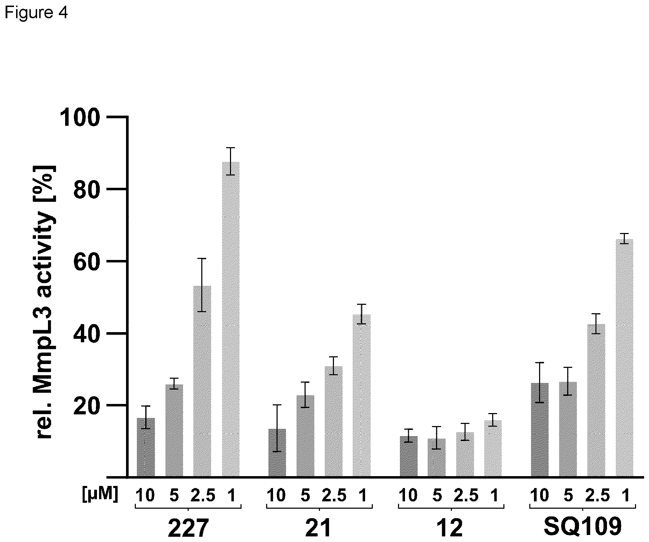

The known MmpL3 inhibitor SQ109 was included as a positive control, reducing the transporter activity to 26% at a concentration of 10 µM (Figure 4). Importantly, compound 227 reduced MmpL3 activity to 16% at 10 µM with high accumulation of TMM, validating this transporter as an antibiotic target. Compounds 21 and 12 with improved MICs against *Mycobacterium tuberculosis* H37Ra effectively inhibit MmpL3 (Figure 4). The Assay was performed in biological replicates (n=3), and the activity was calculated from the ratio of TDM to TMM and then normalized to a DMSO control.

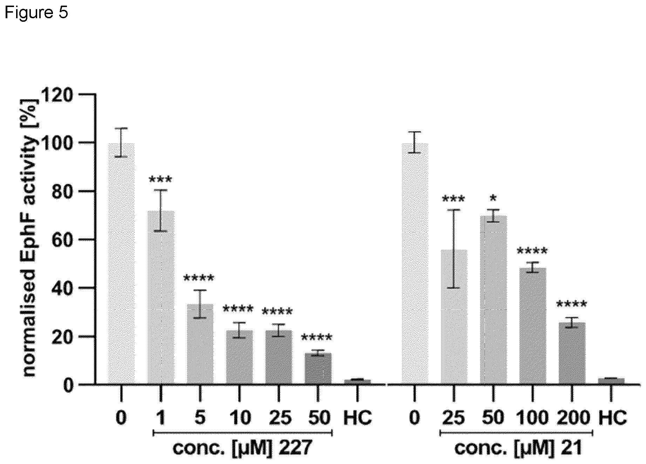

Experiments addressing mycobacterial EpH were performed to validate the target engagement of the compounds according to the invention. EpH employs epoxystearic acid as a substrate. Enzymes EphD and EphF were cloned, expressed, and purified. Functional EphF was successfully expressed allowing the establishment of an *in vitro* activity assay. Protein was pre-treated with compound or DMSO before adding 9-10-cis epoxystearic acid. After 15 minutes, the reaction was quenched with chloroform, the stearic acids were extracted, and taurocholic acid was added as an internal standard. The resulting 9,10-dihydroxystearic acid was relatively quantified by LC-MS/MS. A heat control (HC) of heat-denatured EphF was included to monitor the background hydrolysis of the epoxide. Statistical significance of inhibition (compared to DMSO) was calculated using ordinary one-way ANOVA (* = p < 0.05; *** = p < 0.001; **** = p < 0.0001, n=5). Compound 227 showed strong inhibition even at a 10-fold excess of compound compared to the enzyme. In addition, compound 21 effectively inhibited EphF (Figure 5).

### 4.1 Properties of compounds 21 and 22 vis-à-vis compound 227

Hydrophobicity is expressed in LogP values. Compound 227 has a CLogP of 5.2 and a snapshot PK experiment in mice confirmed poor bioavailability. It was shown that compound 21 has a MIC of 0.8 µM and a CLogP of 3.9, i. e. a lower hydrophobicity than compound 227.

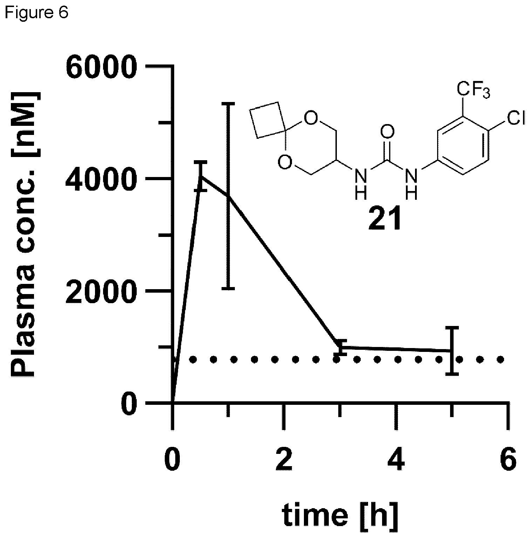

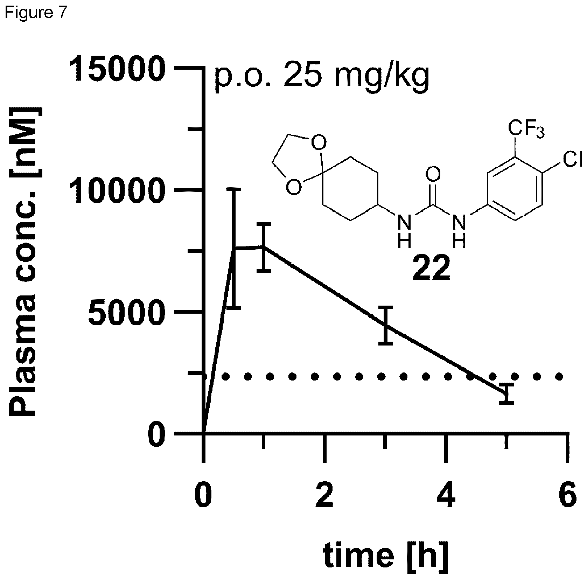

Compound 21 is 2 to 4-fold antibiotically more active than compound 227. To select an effective compound for *in vivo* applications, snapshot PK studies with both compounds, 21 and 22, were determined. Compounds were dosed orally at 25 mg/Kg in 95% (20%) Solutol HS15, 5% DMA, and the plasma concentration was measured over time (n=2). MIC against MTB H37Ra is indicated by the dotted line. (Figure 6 and Figure 7). Compound 21 showed a sub-micromolar MIC and displayed no pronounced cytotoxicity against human cells in a biologically relevant range (IC₅₀ = 71 µM) in MTT assays.

Compound 21 was therefore selected for dose escalation studies.

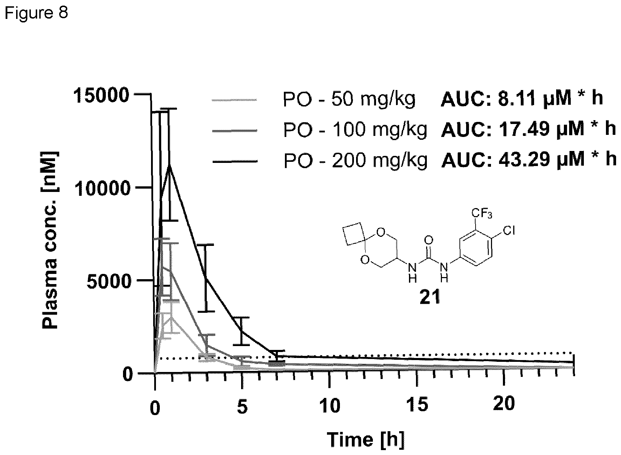

Dose escalation oral PK profiling of compound 21 was performed as described above. Results for 50, 100, and 200 mg/kg in a suspension with 20% Solutol HS15 are given in Figure 8.

The compound was well tolerated, with a dose-dependent increase in the plasma concentration. The bioavailability was slightly decreased compared to the snapshot PK analysis, probably due to a different formulation. Compounds were dosed orally in 20% Solutol HS15 on day 1 and on day 3 (n=3). Time over MIC was about 7 h for a 200 mg/kg dose with an area under the curve (AUC) of 43 µM*h, a maximum concentration at Tₘₐₓ = 1 h, and a half-life of about 2.5 - 3 h, depending on the dose (Figure 8).

## Claims

1. A compound comprising a residue consisting of a first molecular ring and a second molecular ring connected to each other via one common atom which compound has a structure according to formula I 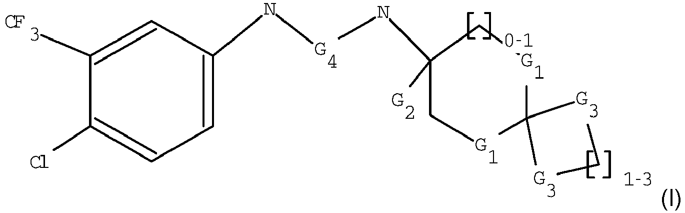 wherein
G₁ is independently selected from a group consisting of C, O, N, and S,
G₂ is independently selected from a group consisting of C and H,
G₃ is independently selected from a group consisting of C, O, N and S and
G₄ is independently selected from a group consisting of C=O, CH₂, C=S and C=NH
or
a pharmaceutically acceptable salt, a solvate or a hydrate of said compound.

2. The compound according to claim 1,
wherein,
G₁ is C or O,
G₂ is H,
G₃ is C or O and
G₄ is C=O, C=S, C=NH, in particular C=O.

3. The compound according to claim 1 or 2, wherein, the first molecular ring is a 6-membered molecular ring and/or the second molecular ring is a 4-membered molecular ring, wherein the residue is in particular 5,9-dioxaspiro[3.5]nonan-7-yl or 1,4-dioxaspiro[4.5]decan-8-yl.

4. The compound according to any of the preceding claims,
wherein,
G₁ is O,
G₂ is H,
G₃ is C, and
G₄ is C=O.
or wherein,
G₁ is C,
G₂ is H,
G₃ is O, and
G₄ is C=O.

5. The compound according to any of the preceding claims, wherein said compound is selected from a group consisting of 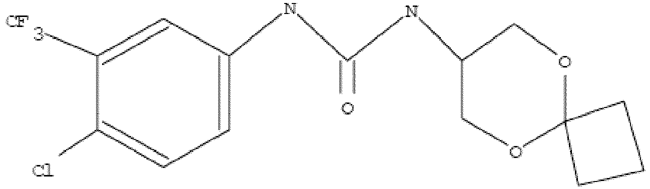 (1-(4-chloro-3-(trifluoromethyl)phenyl)-3-(5,9-dioxaspiro[3.5]nonan-7-yl)urea), a pharmaceutically acceptable salt, a solvate and a hydrate thereof, 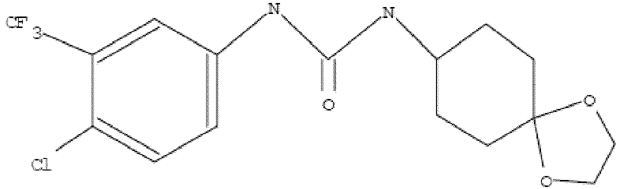 (1-(4-chloro-3-(trifluoromethyl)phenyl)-3-(1,4-dioxaspiro[4.5]decan-8-yl)urea) and a pharmaceutically acceptable salt, a solvate and a hydrate thereof.

6. A pharmaceutical composition comprising the compound of any of the preceding claims and a pharmaceutically acceptable excipient.

7. The pharmaceutical composition according to claim 6 further comprising at least one additional pharmaceutical agent, in particular an antituberculosis agent.

8. The pharmaceutical composition according to claim 7, wherein said antituberculosis agent is selected from the group consisting of isoniazid, rifampicin, pyrazinamide, ethambutol, streptomycin, kanamycin, amikacin, capreomycin, ofloxacin, levofloxacin, moxifloxacin, cycloserine, para-aminosalicylic acid, ethioamide, prothionamide, thioacetazone, clofacimine, amoxicillin with clavulanate, imipenem, linezolid, clarithromycin, and thioridazine.

9. The compound according to any of claims 1 to 5 or the pharmaceutical composition according to any one of claims 6 to 8 for use as a medicament.

10. The compound according to any of claims 1 to 5 or the pharmaceutical composition according to any one of claims 6 to 8 for use in a method for the treatment of a bacterial disease.

11. The compound or the pharmaceutical composition for use according to claim 10, wherein the bacterial disease is a bacterial disease caused by at least one bacteria selected from the group of gram-positive bacteria consisting of *Mycobacterium tuberculosis, multidrug-resistant Mycobacterium tuberculosis, extensively drug-resistant Mycobacterium tuberculosis, bedaquiline-resistant Mycobacterium tuberculosis and clinical isolates thereof, Mycobacterium bovis, Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium, intracellulare, Mycobacterium simiae, Mycobacterium szulgai, Mycobacterium xenopi, Mycobacterium fortuitum, Mycobacterium chelonei, Mycobacterium marinum,* in particular a bacterial disease caused by *multidrug resistant and extensively drug-resistant Mycobacteria.*

12. A kit for preparation of a pharmaceutical composition comprising a compound according to any one of claims 1 to 5 and at least one pharmaceutically acceptable carrier.

13. Use of a compound according to any one of claims 1 to 5 as a disinfectant, wherein any method for treatment of the human or animal body by surgery or therapy is excluded.

14. A method for synthesis of 1-(4-chloro-3-(trifluoromethyl)phenyl)-3-(5,9-dioxaspiro[3.5]nonan-7-yl)urea
comprising at least the following steps:
a) Dissolution of 5,9-dioxaspiro[3.5]nonan-7-amine in a polar aprotic solvent,
b) addition of 1-chloro-4-isocyanato-2-(trifluoromethyl)benzene,
c) stirring of the resulting solution
and either
d1) addition of water and separation of aqueous and organic phase,
e1)washing of the organic phase with water and removal of volatile components of the remaining organic phase by vacuum evaporation until a solid residue is obtained,
f1) dissolution of the residue in a polar aprotic solvent having a dielectric constant of at least 30 at 25 °C and precipitation by addition of water,
g1)filtering of a precipitate obtained in step f) and removal of residual water and
h1) performing a chromatography for obtaining a fraction containing 1-(4-chloro-3-(trifluoromethyl)phenyl)-3-(5,9-dioxaspiro[3.5]nonan-7-yl)urea in an eluent and optionally removing the eluent for obtaining 1-(4-chloro-3-(trifluoromethyl)phenyl)-3-(5,9-dioxaspiro[3.5]nonan-7-yl)urea as a solid product
or
d2) removal of the solvent from the solution and
e2) performing a chromatography for obtaining a fraction containing 1-(4-chloro-3-(trifluoromethyl)phenyl)-3-(5,9-dioxaspiro[3.5]nonan-7-yl)urea in an eluent and optionally removing the eluent for obtaining 1-(4-chloro-3-(trifluoromethyl)phenyl)-3-(5,9-dioxaspiro[3.5]nonan-7-yl)urea as a solid product.

15. A method for synthesis of 1-(4-chloro-3-(trifluoromethyl)phenyl)-3-(1,4-dioxaspiro[4.5]decan-8-yl)urea comprising at least the following steps:
a) Dissolution of 1-chloro-4-isocyanato-2-(trifluoromethyl)benzene in a polar aprotic solvent,
b) addition of 1,4-dioxaspiro[4.5]decan-8-amine,
c) stirring of the resulting solution
and either
d1) filtering of a precipitate formed in step c) and washing of the precipitate for obtaining 1-(4-chloro-3-(trifluoromethyl)phenyl)-3-(1,4-dioxaspiro[4.5]decan-8-yl)urea as a solid product
or
d2) removal of the solvent from the solution and
e2) performing a chromatography for obtaining a fraction containing 1-(4-chloro-3-(trifluoromethyl)phenyl)-3-(1,4-dioxaspiro[4.5]decan-8-yl)urea in an eluent and optionally removing the eluent for obtaining 1-(4-chloro-3-(trifluoromethyl)phenyl)-3-(1,4-dioxaspiro[4.5]decan-8-yl)urea as a solid product.
